# EUROPEAN PATENT SPECIFICATION

(11) **EP 2 976 004 B1**
(45) Date of publication and mention of the grant of the patent: **01.12.2021**
(21) Application number: 14775930.2
(22) Date of filing: 11.03.2014
(51) Int. Cl.: A61B 5/00, H02J 5/00, H02J 50/10, H02J 7/02, A61M 5/14

(54) **INTELLIGENT INDUCTIVE POWER SYSTEM FOR MEDICAL DEVICE AND SYSTEM**
INTELLIGENTES INDUKTIVES STROMVERSORGUNGSSYSTEM FÜR MEDIZINISCHE VORRICHTUNGEN UND SYSTEME
SYSTÈME D'ALIMENTATION INDUCTIF INTELLIGENT POUR DISPOSITIF ET SYSTÈME MÉDICAL

(30) Priority: 14.03.2013 US 201313829744; 14.03.2013 US 201313830869
(43) Date of publication of application: 27.01.2016
(73) Proprietor: CareFusion 303, Inc., San Diego, CA 92130 (US)
(72) Inventor: FERN, David, George, San Diego, CA 92130 (US); HALBERT, Donald, San Diego, CA 92130 (US); BLOOM, Mark, Patrick, San Diego, CA 92130 (US); CHAUHAN, Jeet, San Diego, CA 92130 (US); BORGES, Gregory, San Diego, CA 92130 (US); CARLISLE, Thomas, John, San Diego, CA 92130 (US); CLARKE, Chris, San Diego, CA 92130 (US); DANIEL, Pascal, San Diego, CA 92130 (US); FREARSON, Timothy, Charles, San Diego, CA 92130 (US); NESTERENKO, Igor, V., San Diego, CA 92130 (US); ROBERTS, Stephen, Daniel, San Diego, CA 92130 (US); VIK, Daniel, Hans, San Diego, CA 92130 (US); WILKINSON, James, Patrick, San Diego, CA 92130 (US)
(74) Representative: Molnia, David
(86) International application number: PCT/US2014/023754
(87) International publication number: WO 2014/159462

(56) References cited:
- EP-A2- 1 684 396
- EP-A2- 2 560 116
- WO-A2-2005/050524
- WO-A2-2008/017041
- WO-A2-2012/007942
- RU-C2- 2 336 906
- RU-C2- 2 437 168
- US-A1- 2008 269 716
- US-A1- 2009 106 567

## Description

### TECHNICAL FIELD

The subject matter described herein relates to an intelligent inductive power system for medical device and system.

### BACKGROUND

Medical device systems may utilize a plurality of different medical devices that are distinct stand-alone or independent medical devices. For example, some conventional infusion pumping systems may include up to about four functionally distinct stand-alone infusion pumps. Conventional infusion pumps are typically stand-alone complex devices that are only able to provide independent complex infusion functions. As such, coordination or control of the devices collectively is complex and difficult. Moreover, purchasing of the complex stand-alone devices can be financially burdensome.

Further, hospitals using each of several different models of pumps, each employing distinct user interfaces, makes both learning and practicing their operation more time consuming with risk of error elevated. For instance, there may be pumps for syringe, large volume, patient controlled analgesia, anesthesia and other uses. These difficulties can be compounded when there are other medical devices being used for patient care including various types of vital sign monitors and the like.

Moreover, medical devices require a high level of reliability and serviceability.
From EP 2 560 116 A2 a docking station is known having recesses to receive a plurality of BMUs, each recess having a port for power and/or data coupling with the BMU. WO 2008/017041 discloses a charging base to provide an interface with a bottom surface of a medical device. Furthermore, EP 1 684 396 A2 discloses charging trays for medical devices based on inductive coils and having recesses with a predefined geometry.

### SUMMARY

The present invention is defined by the independent claim 1, defining a system. Preferred embodiments are derivable from the dependent claims 2-15.

The invention is set out in the appended set of claims. The embodiments and/or examples of the following description which are not covered by the appended claims are considered as not being part of the present invention.

The subject matter described herein provides many advantages. For example, the current subject matter provides enhanced usability for clinicians both with regard to the ease of coupling and decoupling medical device modules from the system and in connection with the various user interfaces provided by the system. The current subject matter also provides enhanced mobility with regard to the movement of medical device modules to be used with other inductive backplanes, with other systems, and stand-alone. Furthermore, the current subject matter is advantageous in that it enables contextual operation of medical device modules thereby increasing safety. Still further, the current subject matter is advantageous in that a clinician can scale the number of utilized medical device modules as may be required during the course of treatment for a patient. In addition, the current subject matter is advantageous in that it provides a common unified user interface which, in turn, provides enhanced usability as compared to individualized user interfaces on each type of medical device.

### DESCRIPTION OF DRAWINGS

FIG. 1 is a diagram illustrating a modular medical device system;
FIG. 2 is a diagram illustrating a modular medical device system in a clinical setting;
FIG. 3 is a logic diagram illustrating a medical device module;
FIG. 4 is a diagram illustrating a modular medical device system with a backplane extension;
FIG. 5 is a diagram illustrating a compact modular medical device system;
FIG. 6 is a diagram illustrating a modular medical device system without an optical communications sub-system;
FIG. 7 is a diagram illustrating a compact modular medical device system without an optical communications sub-system;
FIG. 8 is a diagram illustrating a computing landscape including a modular medical device system;
FIG. 9 is a first process flow diagram illustrating a method of operation of a modular medical device system;
FIG. 10 is a second process flow diagram illustrating a method of operation of a modular medical device system; and
FIG. 11 is a logic diagram illustrating an exemplary embodiment of an intelligent inductive power system.

Like reference symbols in the various drawings indicate like elements.

### DETAILED DESCRIPTION

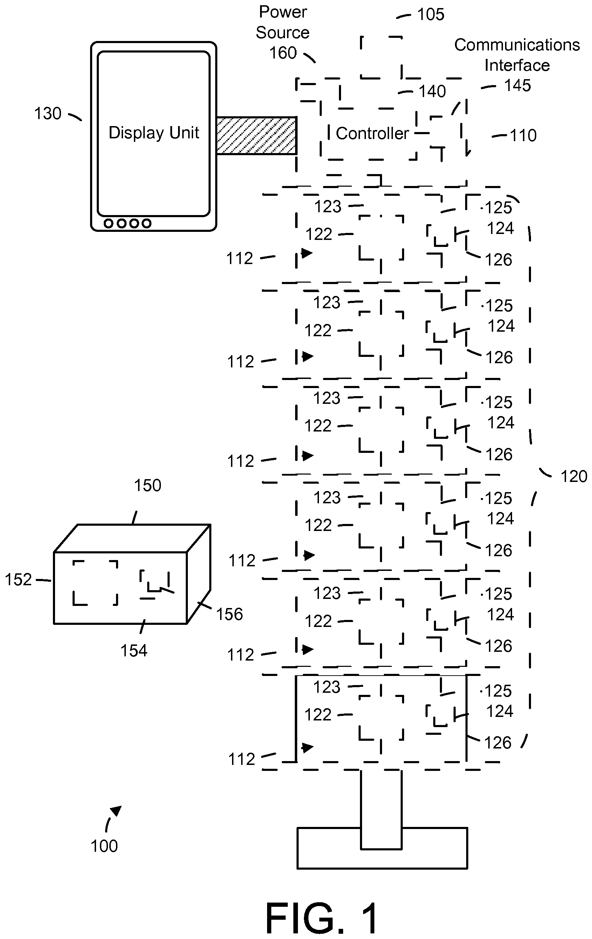

FIG. 1 is a diagram 100 illustrating a modular medication device system 110. The system 110 comprises a backplane 120 that can be mounted on a pole 105. The backplane 120 can mechanically couple to and secure one or more medical device modules 150 along each of a series of pre-defined mounting seats 112. In some variations, each of the mounting seats 112 are uniform in size and spacing, while in other variations different sizing and/or spacing can be used to accommodate medical device modules 150 having different exterior dimensions. In addition, the mounting seats 112 can be arranged along a single axis (e.g., a vertical axis as illustrated, etc.) or they can be arranged along two or more axes. The mounting seats 112 can each have one or more mechanical elements to detachably affix the medical device modules 150 to the backplane 120.

In addition to allowing the medical device modules to be affixed to the system, the backplane 120 can provide non-contact inductive power to one or more medical device modules 150. The backplane 120 can, for each mounting location, comprise an inductive transmitter 122 for non-contact powering of a medical device module 150. A corresponding inductive receiver 152 on the medical device module 150 can, when the medical device module 150 is affixed to the mounting seat 112, be inductively coupled with inductive transmitter 122 of backplane 120. In general, energy is sent via an inductive (magnetic) coupling between inductive transmitter 122 and inductive receiver 152. As a result, there is a wireless (no galvanic contact) energy transfer between inductive backplane 120 and medical device module 150. Moreover, an electrical galvanic connector, as is typical for powering conventional medical devices, is not required to provide power to medical device module 150. Use of non-contacting energy transfer avoids metallic contacts between medical device module 150 and a power source which may be damaged, require special cleaning and pose risk of electrical heating, smoke or fire. Each inductive transmitter 122 can be coupled to an induction bus 123 which in turn is connected to a power source 160 (e.g., a wired connection to an outlet, a battery, etc.) to enable the inductive coupling of each inductive transmitter 122.

The backplane 120 can also provide an optical communications interface to one or more medical device modules 150 via respective optical communications ports 124 and optical transceivers 126 corresponding to each mounting seat 112. The medical device modules 150 can have corresponding optical communications ports 154 and optical transceiver 156 which can be optically aligned with the optical communication port 124 on the backplane 120 when the medical device module 150 is affixed to the backplane 120 so that a bi-directional data feed can be established between the optical transceivers 126, 156. Such data can relate to a variety of aspects including, but not limited to, data characterizing operation of the medical device module 150, data for controlling (e.g., modifying, monitoring, etc.) one more attributes of the medical device module 150 (e.g., software updates, configuration updates, asset locations, status information, historical data, patient information, patient treatment parameters, medication administration information, etc.), and the like. Stated differently, the data exchanged via the optical transceivers 126, 156 can comprise any data generated or otherwise used by a medical device module 150 or a caregiver using same. The data transmitted to the backplane 120 can be consumed locally by the system 110 and/or it can be transmitted to one or more remote systems / devices coupled to the system 110 via a wired or wireless communications link. The optical data transceivers 126, 156 can be infrared (IR) data transceivers such that optical data 146 is propagated by IR light as the transmission medium. The optical data transceivers can be coupled to a communications bus 125 that in turn is coupled to a communications interface 142. The communications interface 142 can, in turn, be coupled to the control unit 140. In addition or in the alternative, a second communications interface 144 can provide an outward interface for the modular medical device system 110 that provides a wired or wireless connection to other devices and/or networks. It will be appreciated that any number of communications interfaces can be used, including one communications interface for each optical data transceiver 126 / seat 112.

The control unit 140 can be hardware, software, or a combination of both. For example, the control unit 140 can be a specially designed hardware module including at least one data and memory with specialized software used to control any aspect of a medical device module 150 coupled to the system 110. In other cases, the control unit 140 can be a software module (or series of modules) used to control any aspect of a medical device module 150 coupled to the system 110. As used herein, unless otherwise specified, the term control shall relate to any type of data exchange with a medical device module 150 by the control unit 140 including data generated by a medical device module 150 and data used by a medical device module 150 (software updates, power state changes, etc.). For example, the control unit 140 can be used to selectively wake up medical device modules 150 coupled to the inductive backplane 120 from a sleep state. Furthermore, the control unit 140 can be coupled to one or more remote computing systems (via the communications interface 144) to allow for the remote control of the medical device modules 150.

Each mounting seat 112 can include a shelf with dove tail features extending from a housing of the system 110. Each medical device module 150 can include a latch mechanism on a top rear edge that affixes to the housing of the system 110. The latch mechanism can reduce load on the shelf and can cause the medical device module 150 to rotate back into contact with the system 110 under load (rather than deflect away from it). This arrangement can help insure that the inductive transmitter 122 is positioned properly and secured in relation to the inductive receiver 152.

Each mounting seat 112 can include a proximity sensor 127 that can detect the presence of a medical device module 150. The proximity sensors 127 can be optical, electric, electro-mechanical, and/or mechanical devices. For example, the proximity sensors 127 can comprise a Hall effect sensor and/or a mechanical switch. The presence of a medical device module 150 can be used to initiate, for example, inductive powering by the corresponding inductive transmitter 122 and/or communications via the communications interface 142. The proximity sensor 127 can also indicate an alarm condition when a medical device module 150 is not completely secured so that appropriate actions can be taken.

Medical device module 150 can be any medical device that is compatible for scalability in a modular medical device system. For instance, the modular medical device system 110 can utilize one or more medical device modules 150 depending on the functionality that is needed for proper care of a patient. Moreover, a modular medical device system 110 can be scaled up to incorporate additional medical device modules 150 and also scaled down by removing medical device modules 150.

For example, if patient care requires only one infusion pump, then the modular medical device system 110 can include a single affixed infusion pump. Moreover, if patient care requires two infusion pumps, then the modular medical device system 110 can be scaled up to include an affixed additional infusion pump.

Medical device modules 150 can include, but is not limited to, an infusion pump (e.g., a large volume pump (LVP), a syringe pump), a patient-controlled analgesia (PCA) system, a vital signs monitor (VSM) (e.g., an SpO2 sensor, an EtCO2 sensors, cardiac output monitors, gastric tonometers, etc.), a bedside blood analyte analyzer (e.g. blood glucose), an Auto-ID module (barcode scanner and RFID), and other devices which measure physiological parameters associated with a patient and/or assist with the clinical care of the patient (and such medical device modules 150 may not necessarily measure physiological parameters of the patient).

Modular medical device system 110 can also comprise a display unit 130 that provides a unified interface that characterizes the operation of various medical device modules 150 coupled to the backplane 120. The display unit 130 can comprise a touch screen interface that allows a user to selectively view and alter performance parameters / metrics of individual medical device modules 150, concurrently view performance parameters / metrics of multiple medical device modules 150, and additionally orchestrate complex sequences of infusions / operations from multiple medical device modules 150. The display unit 130 can be affixed to an outer housing of the modular medical device system 130 / inductive backplane 120 by a tilt and swivel arm mount that allows the display unit to be moved on different sides of the system 110 and/or to change varying positions (to accommodate different positions / heights of caregivers).

The display unit 130 can include a speaker to provide audio cues relating to various aspects of medical device modules 150 (in other versions the speaker is located elsewhere in the system 110). When the medical device modules 150 are coupled to the backplane 120, audio cues such as alarms for such medical device modules 150 can be delegated so that the system 110 handles the alarms whether via an audio and/or visual cue in the display unit 130 or by an audio cue generated elsewhere in the system 110. In some cases, some alarms can be still be handled by a medical device module 150 while other alarms are handled by the system 110.

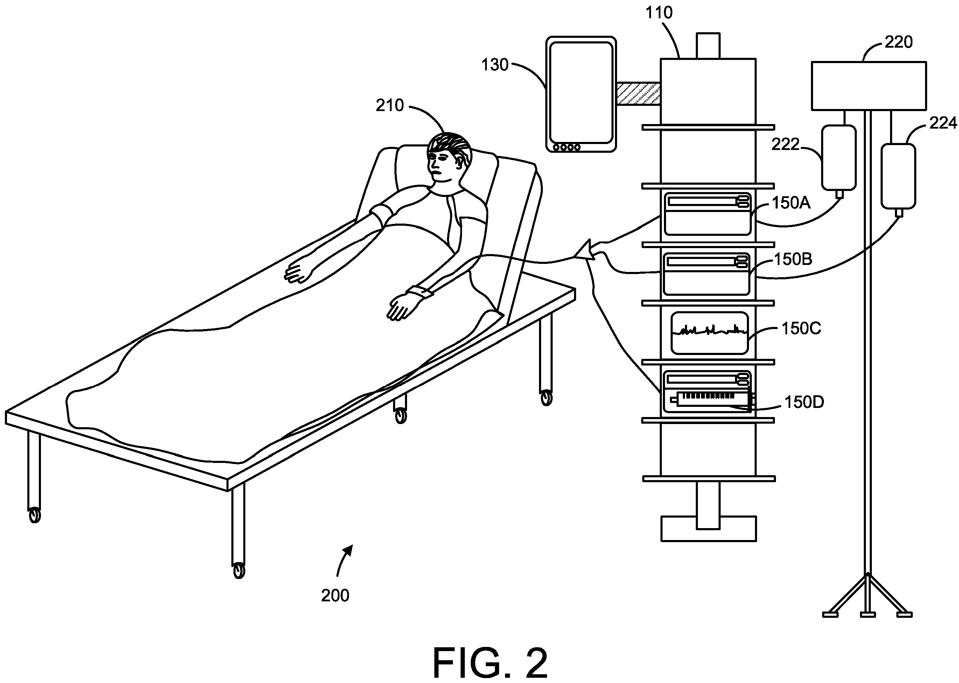

FIG. 2 is a diagram 200 illustrating a modular medical device system 110 in a clinical setting. In particular, in this view, the modular medical device system 110 is coupled to two infusion pumps 150A, 150B, a vital signs monitor 150C, and a syringe pump 150D. The infusion pumps 150A and 150B are respectively fluidically coupled to two fluid / medication containers 222, 224 suspended from an IV pole 220. In addition, each of the infusion pumps 150A and 150B and the syringe pump 150D are fluidically coupled to an IV catheter inserted into a patient 210 so that the corresponding fluids can be delivered to the patient. The modular medical device system 110 monitors and/or controls how fluids from the respective sources 150A, 150B, 150D are delivered to the patient 210. It will be appreciated that varying numbers of medical device modules 150 can be utilized depending on the particular condition of and/or treatment the patient 210.

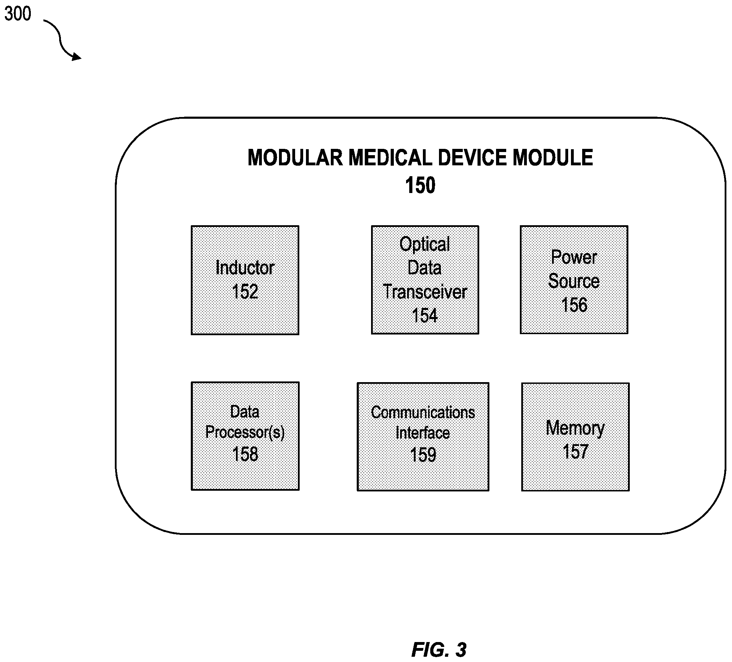

FIG. 3 is a logic diagram 300 of a medical device module 150. With FIG. 3, each medical device module 150 can include a secondary power source 156 such as a battery or a wired connection to an external power source. For example, the secondary power source 156 can power medical device module 120 when inductive receiver 152 is unable to power medical device module 150. In one scenario, medical device module 150 is an infusion pump that is associated with (and fluidly communicating with) a patient. If the patient is moved to another location (e.g., to an x-ray room which is away from inductive backplane 110), then medical device module 150 must also move with the patient away from inductive backplane 120 in order to continue the current infusion without interruption. Upon a certain distance from inductive backplane 120, inductive receiver 152 cannot be energized by inductive backplane 120 and therefore cannot power medical device module 150. In other cases, the inductive backplane 120 may be turned off or operating in a mode not allowing the inductive receiver 152 to be energized. Accordingly, power source 156 (which may also be charged through inductive receiver 152) is able to provide the requisite power for medical device module 150. In one variation, the power source 156 is a battery that can keep medical device module 150 operational in a range of about two to four hours.

Each medical device module 150 can also include memory 157 and at least one data processor 158. The memory 157 can store instructions for execution by the at least one data processor 158 for use, for example, in the operation of the medical device module in a clinical setting. The memory 157 can also store data relating to the operation of the medical device module such as data characterizing how the medical device module 150 is used and parameters relating to same (e.g., number of hours operated, thresholds for alerts, etc.), performance and status information, as well as other aspects relating to the use of such medical device module 150 such as patient data, medication administration data, patient treatment parameters, etc.. It is noted that when a medical device module 150 is reattached to the prior inductive backplane or a different inductive backplane, information required to continue the infusion stored in memory 157, without interruption, can be transmitted from the medical device module 150 to the backplane (and to the control unit 140).

Each medical device module 150 can also comprise an additional communications interface 159 other than the optical data transceiver 154 (in some variations the optical data transceiver 154 may not form part of the medical device module 150 and so the communications interface 159 may be the only gateway for communication outside of the medical device module 150). This communications interface 159 can be fixed and/or wireless and be used to communicate to computer networks and peer-to-peer pairing with other devices when the medical device module 150 is not coupled to the backplane 120.

In some implementations, the communications interface 159 can be used in addition or instead of the optical data transceiver 154 when the medical device module 150 is coupled to the backplane 120. For example, the medical device module 150 can be seated on the backplane 120 but not have an optical data transceiver. In such a scenario, the communications interface 159 can wirelessly communicate with the control unit 140 of the modular medical device system 110 so that the operation of the medical device module 150 can be monitored and/or controlled by the modular medical device system 110 (whether or not the medical device module 150 is seated). Various types of wireless communications can be used (for this and other aspects described herein) such as short distance protocols such as BLUETOOTH, near field communication (NFC), WiFi, ZIGBEE, and the like.

As noted above, the system 110 comprises a control unit 140 (which in turn can comprise at least one data processor and memory for storing instructions for execution by the at least one data processor and/or data characterizing or otherwise relating to the operation of medication device modules 150). The control unit 140 can act to individually monitor and/or control the operation of the medical device modules 150 affixed to the backplane 120 such that the functionality of the medical device modules 150, alone and/or in combination are increased. In some cases, the control unit 140 can orchestrate the operation of multiple medical device modules 150. For example, certain sequences of operation and/or concurrent operation can be defined amongst the medical device modules 150. Such an arrangement can permit, for example, coordinated infusion from different fluid sources. Some medical device modules 150 can have the ability to function fully independent of the control unit 140 for the purpose of basic operations. However, the modules acquire more complex abilities and functionality when operating under the command and coordination of the controller.

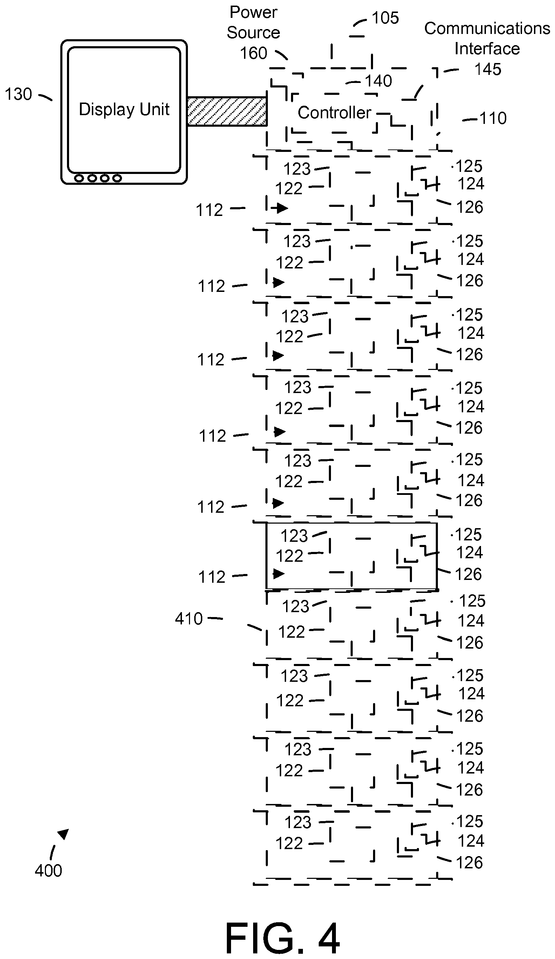

FIG. 4 is a diagram 400 that illustrates a backplane extension 410. The backplane extension 410 provides additional seats 112 to which medical device modules 150 can be coupled. The backplane extension 410 can be coupled physically and/or electrically to the system 110 so that any medical device modules 150 coupled to the backplane extension 410 can be coupled to the control unit 140 and function in a similar manner to the medical device modules 150 coupled to the backplane 120. Stated differently, backplane extension 410 can function similarly to the inductive backplane 120. It will be appreciated that the backplane 120 and the backplane extension 410 can each be configured to seat any number of medical device modules 150 as may be desired.

In some clinical settings, multiple backplane extensions 410 and/or backplanes 120 can be utilized to serve a single patient. With such an arrangement, each inductive backplane 120 and backplane extension 410 can have local communication with the other inductive backplanes 120, 410 serving the same patient to provide coordination of functionality and data. The communication can be wired and/or wireless using, for example, short range digital radio technology, WiFi, optical data transceivers, BLUETOOTH, ZIGBEE, NFC, and the like.

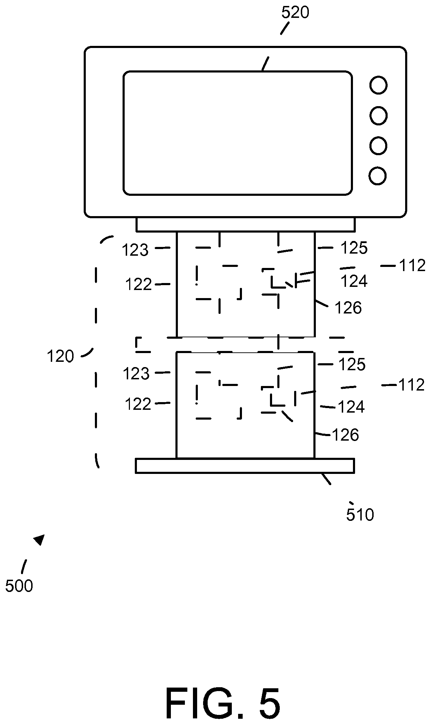

FIG. 5 is a diagram 500 that illustrates a variation of a modular medical device system 510 that is more compact. This modular medical device system 120 can, for example, include fewer seats 112 to which medical device modules 150 can be affixed, than the modular medical device system 110 (illustrated in FIG. 1) to allow it to sit on a table top or other flat surface. In addition, the display 520 can be placed immediately above the medical device modules 150 to further decrease the footprint of the modular medical device system 520.

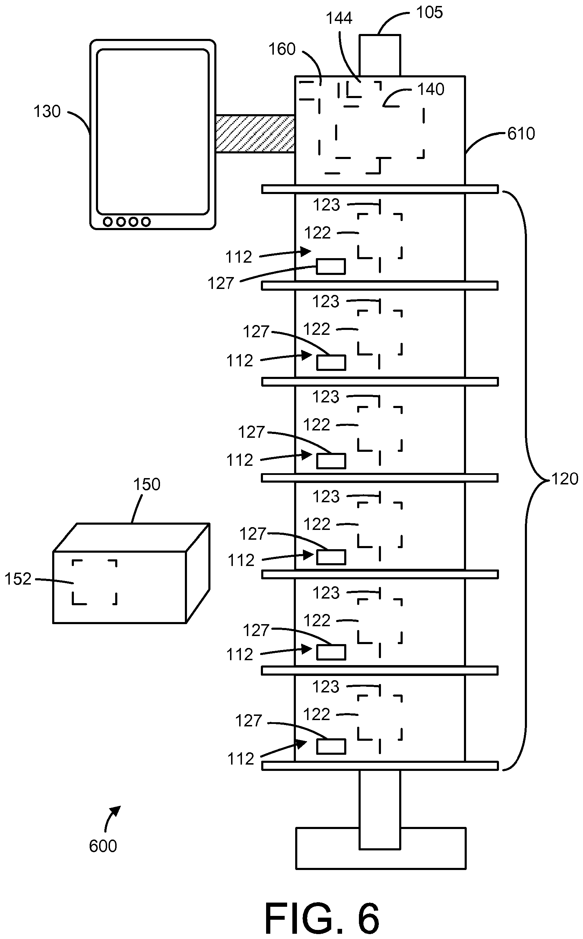

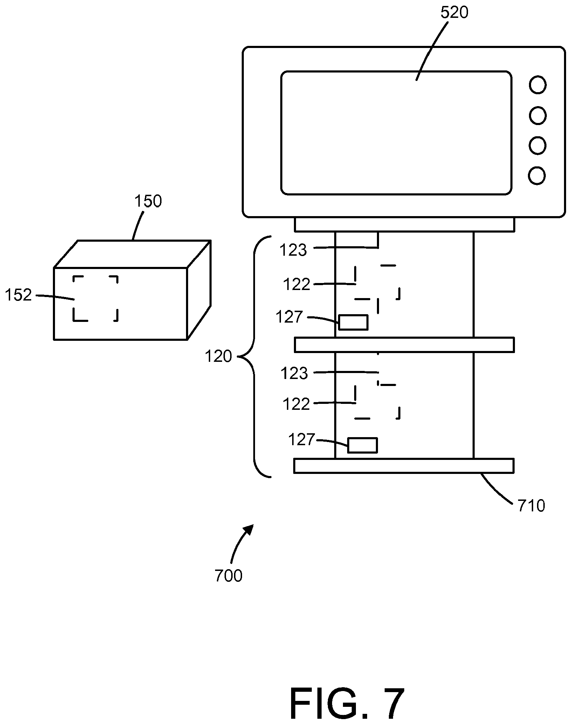

FIG. 6 is a diagram 600 of a modular medical device system 610 and FIG. 7 is a diagram 700 of a compact modular medical device system 710 that respectively illustrate alternative implementations to the modular medical device system 110 of FIG. 1 and the compact modular medical device system 510 of FIG. 5. With both of these variations, communications with medical device modules 150 are effected using a communications protocol different from the optical sub-systems (124, 125, 126, 142). For example, in some variations, the inductive transmitters 122 and inductive bus 123 can be used to exchange data with the inductive receiver 152 to effect a near field magnetic induction communication system. Such an arrangement can provide a short range wireless physical layer that communicates by coupling a tight, low-power, non-propagating magnetic field between the inductive transmitter 122 and the inductive receiver 152. The transmitter coil in the inductive transmitter 122 can modulate a magnetic field which is measured by the inductive receiver 152 in another device. It will be appreciated that the communications are bi-directional and as such, the inductive receiver 152 can also transmit data to the inductive transmitter 122.

In other variations, the modular medical device systems 610, 710 can communicate and exchange data with the medical device modules 150 via a wireless communications protocol including, but not limited to short range digital radio technology, WiFi, optical data transceivers, BLUETOOTH, ZIGBEE, NFC, and the like.

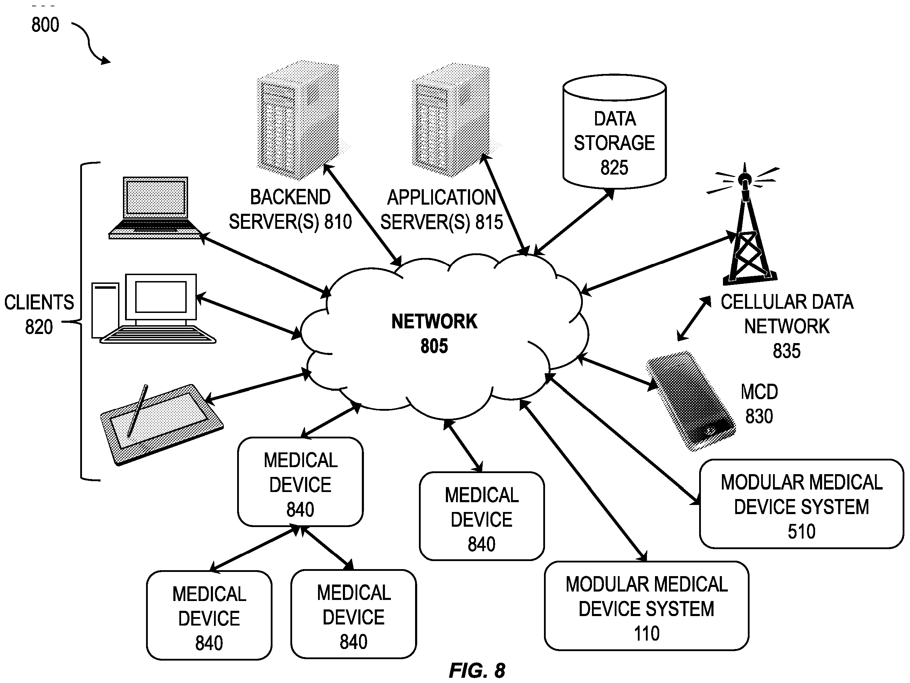

FIG. 8 is a system diagram illustrating a computing landscape 800 within a healthcare environment such as a hospital that includes modular medical device systems 110, 510. Various devices and systems, both local to the healthcare environment and remote from the healthcare environment, can interact via at least one computing network 805. This computing network 805 can provide any form or medium of digital communication connectivity (i.e., wired or wireless) amongst the various devices and systems. Examples of communication networks include a local area network ("LAN"), a wide area network ("WAN"), and the Internet. In some cases, one or more of the various devices and systems can interact directly via peer-to-peer coupling (either via a hardwired connection or via a wireless protocol such as Bluetooth or WiFi). In addition, in some variations, one or more of the devices and systems communicate via a cellular data network.

The modular medical device systems 110, 150 can include at least one communications interface that can access the computing network 805 either via a fixed wired connection or via a wireless connection (via, for example, one or more access points). In addition, the modular medical device systems 110, 150 can also couple to other components within the computing landscape 800 via direct wired or wireless peer-to-peer coupling (not shown). Furthermore, in some cases, one or more of the modular medical device systems 110, 510 can be self-contained and is not connected to any other devices or networks. The modular medical device systems 110, 510 can transmit data via the computing network 805 to any of the other components within the landscape 800 that characterizes the medical device modules 150. In addition, the modular medical device systems 110, 510 can receive data from the computing network 805 relating to monitoring and in some cases controlling one or more attributes of the medical device modules 150 (e.g., software updates, configuration updates, historical data, status information, assets location, patient information, etc.).

In particular, aspects of the computing landscape 800 can be implemented in a computing system that includes a back-end component (e.g., as a data server 810), or that includes a middleware component (e.g., an application server 815), or that includes a front-end component (e.g., a client computer 820 having a graphical user interface or a Web browser through which a user may interact with an implementation of the subject matter described herein), or any combination of such back-end, middleware, or front-end components. A client 820 and server 810, 815 are generally remote from each other and typically interact through the communications network 805. The relationship of the clients 820 and servers 810, 815 arises by virtue of computer programs running on the respective computers and having a client-server relationship to each other. Clients 820 can be any of a variety of computing platforms that include local applications for providing various functionality within the healthcare environment. Example clients 820 include, but are not limited to, desktop computers, laptop computers, tablets, and other computers with touch-screen interfaces. The local applications can be self-contained in that they do not require network connectivity and/or they can interact with one or more of the servers 810, 815 (e.g., a web browser).

A variety of applications can be executed on the various devices and systems within the computing landscape such as electronic health record applications, medical device monitoring, operation, and maintenance applications, scheduling applications, billing applications and the like. As another example, the applications can comprise a collection of enterprise-based applications that provide dose error reduction software (DERS) for the modular medical device systems 110, 150 incorporates a role-based view of infusion data, provides a comprehensive platform for connectivity to external hospital applications, and enables directed maintenance and calibration activities for devices, storage of clinical and device history, etc. As a further example, the applications can provide for remote alarms management and/or asset tracking for medical device modules 150 coupled to one or more of the modular medical device systems 110, 150.

The network 805 can be coupled to one or more data storage systems 825. The data storage systems 825 can include databases providing physical data storage within the healthcare environment or within a dedicated facility. In addition, or in the alternative, the data storage systems 825 can include cloud-based systems providing remote storage of data in, for example, a multi-tenant computing environment. The data storage systems 825 can also comprise non-transitory computer readable media.

Mobile communications devices (MCDs) 830 can also form part of the computing landscape 800. The MCDs 830 can communicate directly via the network 805 and/or they can communicate with the network 805 via an intermediate network such as a cellular data network. Various types of communication protocols can be used by the MCDs 830 including, for example, messaging protocols such as SMS and MMS. In some cases, the MCDs 830 can receive alerts generated from the operation of the medical device modules 150 coupled to the backplane 120 and/or they can otherwise be used to monitor the operation of such medical device modules 150.

Various types of medical devices 840 can be used as part of the computing landscape 800. These medical devices 840 can comprise, unless otherwise specified, any type of device or system with a communications interface that characterizes one or more physiological measurements of a patient and/or that characterize or are used for the treatment of a patient. In some cases, the medical devices 840 communicate via peer to peer wired or wireless communications with another medical device 840 (as opposed to communicating with the network 805). For example, the medical device 840 can comprise a bedside vital signs monitor that is connected to other medical devices 840 (and/or the modular medical device system 110, 510), namely a wireless pulse oximeter and to a wired blood pressure monitor. One or more attributes of the medical devices 840 can be locally controlled by a clinician, controlled via a clinician via the network 805, and/or they can be controlled by one or more of a server 810, 815, a client 620, a MCD 830, and/or another medical device 840 or the modular medical device systems 110, 510.

The computing landscape 800 can provide various types of functionality as may be required within a healthcare environment such as a hospital. For example, a pharmacy can initiate a prescription via one of the client computers 820. This prescription can be stored in the data storage 825 and/or pushed out to other clients 820, an MCD 830, and/or one or more of the medical devices 840 and/or one of the medical device modules 150 forming part of the modular medical device system 110. In addition, the medical devices 840 and the modular medical device system 110 can provide data characterizing one or more physiological measurements of a patient and/or treatment of a patient (e.g., medical device 840 can be an infusion management system, etc.). The data generated by the modular medical device system 110 and the medical devices 840 can be communicated to other medical devices 840, the servers 810, 815, the clients 820, the MCDs 830, and/or stored in the data storage systems 825.

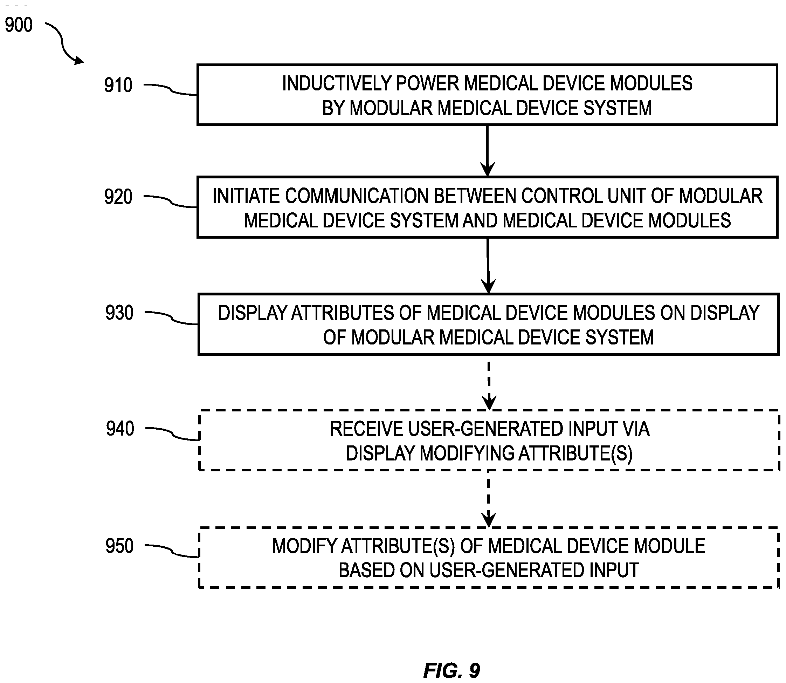

Various methods can be implemented in accordance with the current subject matter. FIG. 9 is a process flow diagram 900 illustrating a method in which, at 910, a modular medical device system inductively powers each of a plurality of medical device modules. The modular medical device system includes an inductive backplane configured to secure and inductively power the plurality of medical device modules, a communications interface, a display, and a control unit. The control unit can control at least one attribute of each medical device module via the communications interface when the medical device module is secured to the inductive backplane. Thereafter, at 920, communications are initiated between each of the medical device modules and the control unit via the communications interface. The display, at 930, then displays one or more attributes characterizing operation of at least one of the medical device modules. In addition, optionally, at 940, user-generated input modifying at least one attribute of at least one medical device module is receiving via the display of the modular medical device system. As a result, at 950, the control unit modifies the at least one attribute for the at least one medical device module as specified by the user-generated input. In other scenarios, one or more attributes of the medical device modules can be modified from a component / source remote from the modular medical device system.

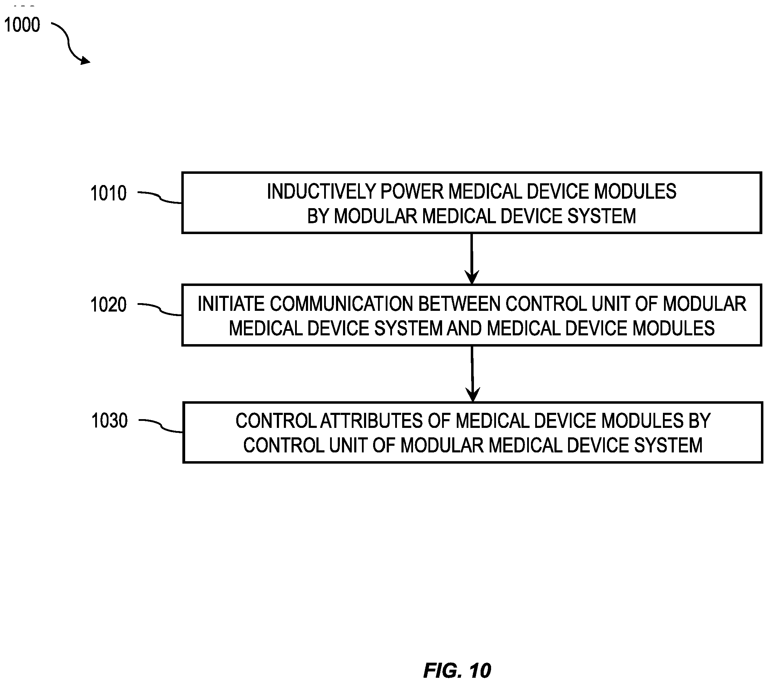

FIG. 10 is a process flow diagram 1000 illustrating a method in which, at 1010, a modular medical device system inductively powers each of a plurality of medical device modules. The modular medical device system includes an inductive backplane configured to secure and inductively power the plurality of medical device modules, a communications interface, a display, and a control unit. The control unit can control at least one attribute of each medical device module via the communications interface when the medical device module is secured to the inductive backplane. Thereafter, at 1020, communications is initiated between each of the medical device modules and the control unit via the at least one communications interface. One or more attributes of at least one of the medical device modules are then, at 1030, controlled by the control unit.

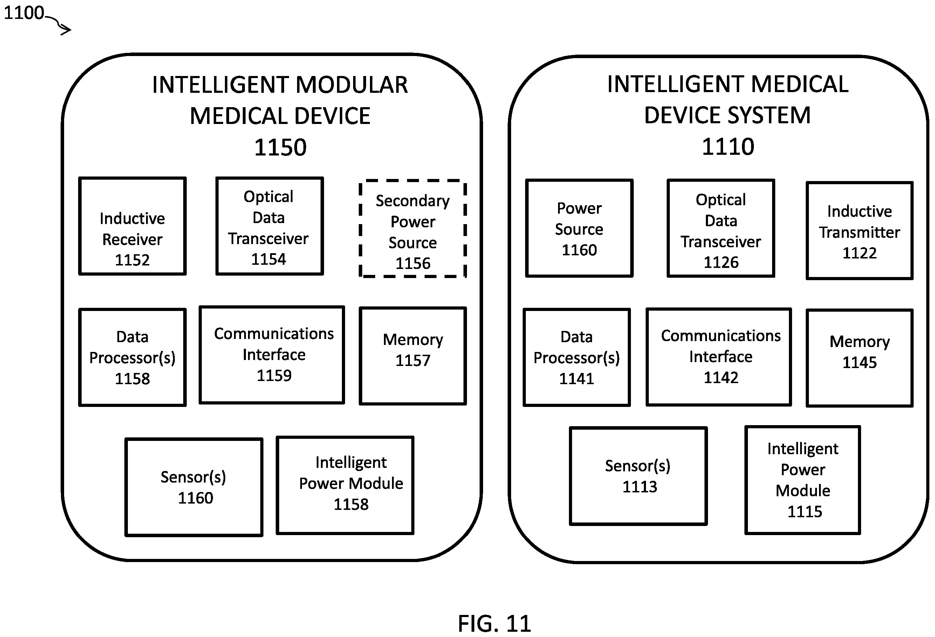

FIG. 11 is a logic diagram 1100 of an intelligent inductive power system. With FIG. 11, an intelligent medical device system 1110 and one or more intelligent medical device modules 1150 can be provided. The intelligent medical device system 1110 can be similar to the medical device system 110 shown in FIGS. 1-7, and can include a power source 1160, optical data transceiver 1126, one or more inductive transmitters 1122, data processor(s) 1141, communications interface 1142, memory 1145, one or more sensors 1113, and intelligent power module 1115. In some implementations, the data processor(s) 1141, memory 1145, and the intelligent power module 1115 may be part of the control unit 140.

Each medical device module 1150 can be similar to the medical device module 150 shown in FIGS. 1-7 and can include inductive receiver 1152, optical data transceiver 1154, data processor(s) 1158, memory 1157, communications interface 1159, one or more sensors 1160, and intelligent power module 1158. The one or more sensors 1160 can include, for example, one or more temperature sensors and/or electrical sensors. In some implementations, the medical device module 1150 can also include optional secondary power source 1156 (e.g. to power the medical device module 1150 when inductive receiver 1152 is unable to power medical device module 1150, or to provide supplemental power to the medical device module 1150).

Memory 1157 of the medical device module 1150 can include instructions for execution by the at least one data processor 1158 for use, for example, in the operation of the medical device module in a clinical setting. Additionally, memory 1157 can also include instructions for execution by the at least one data processor 1158 for use, for example, in implementing the intelligent power module 1158 to monitoring, storing, and/or transmitting one or more parameters/data of inductive receiver(s) 1152 to the medical device system 1110. For example, the parameters/data can include one or more of: inductive controller manufacturer ID, firmware release ID, link efficiency, last error code, device status, input voltage, input current, output power, temperature, and other sensor data. In some implementations, more than one inductive receiver can be provided, and one or more of the above parameters/data can be provided for each inductive receiver 1152. In some implementations, a communication link to the intelligent medical device system 1110 can be provided via a serial bridge to exchange, e.g., the parameters/data and control values over this bridge. In some implementations, the intelligent power module 1158 (e.g. using data processor (s) 1158) may be configured to determine, one or more of, e.g.: a link current, efficiency, and temperature operating state of each inductive receiver 1152 based upon special instructions and feedback loops to implement these features.

In some implementations, the one or more parameters/data of the intelligent medical device module 1150 can be transmitted (e.g. alternative or in addition to the serial bridge) over the optical data transceiver 1154. In some implementations, the one or more parameters/data can be transmitted (e.g. alternative or in addition to the serial bridge and/or the optical data transceiver 1154) using a near field magnetic induction communication system via the inductive receiver 1152 and the inductive transmitter 1122. The one or more parameters/data of the medical device module 1150 can be transmitted in response to one or more queries/interrogations from the medical device system 1110.

In some implementations, each medical device module 1150 can also comprise an additional communications interface other than the optical data transceiver 1154 (in some variations, the optical data transceiver 1154 may not form part of the medical device module 1150 and so the communications interface 1159 may be the only gateway for communication outside of the medical device module 1150). The communications interface 1159 can be fixed and/or wireless and be used to communicate to computer networks and peer-to-peer pairing with other devices when the medical device module 1150 is not coupled to the backplane 120. In some implementations, the communications interface can be used in addition or instead of the optical data transceiver 1154 when the medical device module 1150 is coupled to the backplane 120. For example, the medical device module 1150 can be seated on the backplane 120 but not have an optical data transceiver. In such a scenario, the communications interface 1159 can wirelessly communicate with one or more data processors 1141 of the controller of the modular medical device system 1110 so that the one or more parameters/data can be monitored and/or controlled by the medical device system 1110.

In some implementations, the medical device system 1110 can include memory 1145 which include instructions for execution by the at least one data processor 1141 for use, for example, in receiving the one or more parameters/data from each medical device module 1150. The medical device system 1110 can also include instructions stored in the memory 1145 for execution by the at least one data processor 1141 for implementing one or more features of the intelligent power module 1115, for example, in analyzing the parameters/data from each medical device module 1150, and determining if there has been any failures in the inductive receiver(s) 1152. Additionally, memory 1145 can also include instructions for execution by the at least one data processor 1141 for use, for example, in implementing the intelligent power module 1158 to monitoring, storing, and/or transmitting one or more parameters/data of inductive transmitter(s) 1122. For example, the parameters/data of the inductive transmitter(s) 1122 can include one or more of: inductive controller manufacturer ID, firmware release ID, link efficiency, last error code, device status, input voltage, input current, output power, temperature, and other sensor data. In some implementations, the intelligent power module 1115 (e.g. using data processor (s) 1141) may be configured to determine, one or more of, e.g.: a link current, efficiency, and temperature operating state of each inductive transmitter 1122 based upon special instructions and feedback loops to implement these features.

In some implementations, the parameters/data of the inductive receiver(s) 1152 and/or transmitter(s) 1122 can be analyzed by the at least one data processor 1141 (and/or the data processor(s) 1158) to generate one or more service data including, for example, a recommended service interval, one or more error codes, and recommended service action, which can be accessed by a user and/or transmitted to an alert system. For example, if one or more inductive receiver(s) 1152 and/or transmitter(s) have been operating at a temperature above a threshold for a certain time period, this information can transmitted to alert a user (e.g., nurse, technician) of the issue. The service data can include, for example, identification of a specific part failure, identification of the specific module containing the failed part, identification of the failure type and/or error code, etc. By providing specific service data, a user (e.g. technician or nurse) to quickly diagnose where the failure has occurred and which equipment needs replacement and/or repair.

In some implementations, the one or more parameters/data can be used to monitor or assess the performance of the medical device module. In some implementations, the one or more parameters/data can also be used to estimate when preventive maintenance of one or more parts of the medical device module 1150 and/or the medical device system 1110 should be performed.

In some implementations, the intelligent power module 1115 and/or 1158 is configured to track trends of the individual inductive circuits (e.g. including each inductive receiver(s) 1152 and inductive transmitter(s) 1122) to determine if there is a progression over time towards undesirable conditions such as, e.g. higher current and/or temperatures. The intelligent power module 1115 and/or 1158 can also be configured to compare the parameters/data of the inductive transmitter(s) 1122 and/or receiver(s) 1152 against one or more hard limits (e.g. voltage, current, and/or temperature) such that if one or more hard limits have been reached, the associated inductive transmitter(s) 1122 and/or receiver(s) 1152 is shut down.

In some implementations, the intelligent power module 1115 and/or 1158 is configured to provide short term mitigation by, e.g., dynamically adapt the current draw of one or more components of the intelligent medical device module 1150 and/or the intelligent medical device system 1110 for a time period to provide a temporary relief of the operating conditions of the inductive system (e.g. the inductive transmitter(s) 1122 and/or receiver(s) 1152). This may include one or more of, e.g., reducing or eliminating any battery charging current, and reducing the backlight brightness of the display.

One or more aspects or features of the subject matter described herein may be realized in digital electronic circuitry, integrated circuitry, specially designed ASICs (application specific integrated circuits), computer hardware, firmware, software, and/or combinations thereof. These various implementations may include implementation in one or more computer programs that are executable and/or interpretable on a programmable system including at least one programmable processor, which may be special or general purpose, coupled to receive data and instructions from, and to transmit data and instructions to, a storage system, at least one input device (e.g., mouse, touch screen, etc.), and at least one output device.

These computer programs, which can also be referred to programs, software, software applications, applications, components, or code, include machine instructions for a programmable processor, and can be implemented in a high-level procedural and/or object-oriented programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid state memory or a magnetic hard drive or any equivalent storage medium. The machine-readable medium can alternatively or additionally store such machine instructions in a transient manner, such as for example as would a processor cache or other random access memory associated with one or more physical processor cores.

These computer programs, which can also be referred to programs, software, software applications, applications, components, or code, include machine instructions for a programmable processor, and can be implemented in a high-level procedural language, an object-oriented programming language, a functional programming language, a logical programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid state memory or a magnetic hard drive or any equivalent storage medium. The machine-readable medium can alternatively or additionally store such machine instructions in a transient manner, such as for example as would a processor cache or other random access memory associated with one or more physical processor cores.

With certain aspects, to provide for interaction with a user, the subject matter described herein can be implemented on a computer having a display device, such as for example a cathode ray tube (CRT) or a liquid crystal display (LCD) monitor for displaying information to the user and a keyboard and a pointing device, such as for example a mouse or a trackball, by which the user may provide input to the computer. Other kinds of devices can be used to provide for interaction with a user as well. For example, feedback provided to the user can be any form of sensory feedback, such as for example visual feedback, auditory feedback, or tactile feedback; and input from the user may be received in any form, including, but not limited to, acoustic, speech, or tactile input. Other possible input devices include, but are not limited to, touch screens or other touch-sensitive devices such as single or multi-point resistive or capacitive trackpads, voice recognition hardware and software, optical scanners, optical pointers, digital image capture devices and associated interpretation software, and the like.

The subject matter described herein may be implemented in a computing system that includes a back-end component (e.g., as a data server), or that includes a middleware component (e.g., an application server), or that includes a front-end component (e.g., a client computer having a graphical user interface or a Web browser through which a user may interact with an implementation of the subject matter described herein), or any combination of such back-end, middleware, or front-end components. The components of the system may be interconnected by any form or medium of digital data communication (e.g., a communication network). Examples of communication networks include a local area network ("LAN"), a wide area network ("WAN"), the Internet, WiFI (IEEE 802.11 standards), NFC, BLUETOOTH, ZIGBEE, and the like.

The computing system may include clients and servers. A client and server are generally remote from each other and typically interact through a communication network. The relationship of client and server arises by virtue of computer programs running on the respective computers and having a client-server relationship to each other.

## Claims

1. A system (110) comprising:
a plurality of detachable medical device modules (150), and an inductive backplane (120) configured to secure and inductively power the plurality of detachable medical device modules (150) comprising at least one fluid infusion pump (150A, 150B), wherein the inductive backplane comprises a plurality of mounting seats (112), each mounting seat being configured to mechanically secure one of the plurality of medical device modules (150);
the inductive backplane (120) includes, for each mounting seat (112), an inductive transmitter (122) for non-contact powering of one of the plurality of medical device modules (150), wherein a corresponding inductive receiver (152) on the respective medical device module (150) is, when the respective medical device module (150) is affixed to the mounting seat (112), inductively coupled with the inductive transmitter (122) of the inductive backplane (120) so that energy is sent via an inductive coupling between the inductive transmitter (122) and the inductive receiver (152);
a respective communications interface (124) for each mounting seat (112); and
a control unit (140) to control, via the at least one communications interface (124), at least one attribute of each medical device module (150) when the respective medical device module (150) is secured to the inductive backplane (120),
**characterized in that**
each mounting seat (112) includes a shelf with a dove tail feature extending from a housing of the system (110) configured to interact with a corresponding latch mechanism on a top rear edge of the respective medical device module (150) for affixing the respective medical device module (150) to the housing such that in the affixed state the latch mechanism is configured to reduce load on the shelf and to cause the respective medical device module (150) to rotate back into contact with the system (110) under load to insure that the inductive transmitter (122) is positioned properly and secured in relation to the inductive receiver (152).

2. A system as in claim 1, further comprising:
at least one optical data transceiver (126) coupled to the at least one communications interface (124); and
a plurality of first optical data transmission ports (124) along the inductive backplane (120) coupled to the at least one communications interface (124);
wherein each medical device module (150) comprises a second optical data transmission port (154) positioned along an optical path with a corresponding first optical data transmission port when the respective medical device module (150) is secured to the inductive backplane (120)

3. A system as in claim 2, wherein the communications interfaces (124) comprise a communications bus (125) coupled to each optical data transceiver (126).

4. A system as in claim 1, wherein the communications interfaces (124) are coupled to the inductive backplane (120) and the control unit (140) is configured to control, via an induction-based communications protocol, the at least one attribute of each medical device module (150), when the respective medical device module (150) is secured to the inductive backplane (120)

5. A system as in any of the preceding claims, wherein each medical device module (150) does not comprise an electrical galvanic connector.

6. A system as in any preceding claim, wherein the plurality of mounting seats (112) are arranged along a vertical axis of the inductor backplane (120).

7. A system as in any preceding claim, further comprising a proximity sensor (127) corresponding to each mounting seat (112), the proximity sensor (127) being configured to detect presence of one of the plurality of the medical device modules (150) in the corresponding mounting seat (112).

8. A system as in claim 7, wherein the at least one communications interface (124) is configured to initiate communications with one of the plurality of the medical device modules (150) in a particular mounting seat (112) after the corresponding proximity sensor (127) detects the presence of the respective medical device module (150) in the mounting seat (112).

9. A system as in claim 7 or 8, wherein the inductive backplane (120) is configured to commence inductive powering of one of the plurality of the medical device modules (150) in a particular mounting seat (112) after the corresponding proximity sensor (127) detects the presence of the respective medical device module (150) in the mounting seat (112).

10. A system as in any of the preceding claims, further comprising: an auxiliary power source (156) for powering one or more of the plurality of medical device modules (150) that are not inductively powered.

11. A system as in any of the preceding claims, further comprising:
a touch screen display (130); and
an adjustable display arm coupling the touch screen display (130) with the inductive backplane (120).

12. A system as in claim 11, further comprising:
a housing enveloping the inductive backplane (120);
wherein the touch screen display (130) is integrated into an outer surface of the housing.

13. A system as in any of the preceding claims, wherein each of the plurality of detachable medical device modules (150) comprises at least one inductor (1122) and at least a first sensor (1113) configured for sensing at least a first parameter of the at least one inductor (1122).

14. A system as in claim 13, wherein the at least one communications interface (124) includes a serial bridge configured for exchanging the at least one attribute and the first parameter between the control unit (140) and at least one of the plurality of detachable medical device modules (150).

15. A system as in claim 13 or 14, wherein the control unit (140) is configured to reduce power consumption of one or more components of one of the plurality of detachable medical device modules (150), when the first parameter said detachable medical device modules (150) exceeds a first limit.

## Patentansprüche

1. System (110), umfassend:
eine Vielzahl von abnehmbaren Modulen für medizinische Vorrichtungen (150), und
eine induktive Rückwand (120), die konfiguriert ist, um die Vielzahl von abnehmbaren Modulen (150) für medizinische Vorrichtungen, die mindestens eine Fluidinfusionspumpe (150A, 150B) umfassen, zu sichern und induktiv mit Strom zu versorgen, wobei die induktive Rückwand eine Vielzahl von Montagesitzen (112) umfasst, wobei jeder Montagesitz konfiguriert ist, um eines der Vielzahl von Modulen (150) für medizinische Vorrichtungen mechanisch zu sichern;
wobei die induktive Rückwand (120) für jeden Montagesitz (112) einen induktiven Sender (122) zur kontaktlosen Stromversorgung eines der Vielzahl von Modulen (150) für medizinische Vorrichtungen einschließt, wobei ein entsprechender induktiver Empfänger (152) auf dem jeweiligen Modul (150) für medizinische Vorrichtungen, wenn das jeweilige Modul (150) für medizinische Vorrichtungen an dem Montagesitz (112) befestigt ist, induktiv mit dem induktiven Sender (122) der induktiven Rückwand (120) gekoppelt ist, so dass Energie über eine induktive Kopplung zwischen dem induktiven Sender (122) und dem induktiven Empfänger (152) gesendet wird;
eine jeweilige Kommunikationsschnittstelle (124) für jeden Montagesitz (112); und
eine Steuereinheit (140), um über die mindestens eine Kommunikationsschnittstelle (124) mindestens eine Eigenschaft jedes Moduls (150) für medizinische Vorrichtungen zu steuern, wenn das jeweilige Modul (150) für medizinische Vorrichtungen an der induktiven Rückwand (120) gesichert ist,
**dadurch gekennzeichnet,**
**dass** jeder Montagesitz (112) eine Ablage mit einem Schwalbenschwanzmerkmal einschließt, die sich von einem Gehäuse des Systems (110) erstreckt, konfiguriert, um mit einem entsprechenden Verriegelungsmechanismus an einer oberen Hinterkante des jeweiligen Moduls (150) für medizinische Vorrichtungen zusammenzuwirken, um das jeweilige Modul (150) für medizinische Vorrichtungen an dem Gehäuse zu befestigen, so dass in dem befestigten Zustand der Verriegelungsmechanismus konfiguriert ist, um die Last auf die Ablage zu reduzieren und zu veranlassen, dass das jeweilige Modul (150) für medizinische Vorrichtungen unter Last zurück in Kontakt mit dem System (110) rotiert, um sicherzustellen, dass der induktive Sender (122) korrekt positioniert und in Bezug auf den induktiven Empfänger (152) gesichert ist.

2. System nach Anspruch 1, weiter umfassend:
mindestens einen optischen Daten-Sendeempfänger (126), der mit der mindestens einen Kommunikationsschnittstelle (124) gekoppelt ist; und
eine Vielzahl von ersten optischen Datenübertragungsanschlüssen (124) entlang der induktiven Rückwand (120), die mit der mindestens einen Kommunikationsschnittstelle (124) gekoppelt sind;
wobei jedes Modul (150) für medizinische Vorrichtungen einen zweiten optischen Datenübertragungsanschluss (154) umfasst, der entlang eines optischen Pfades mit einem entsprechenden ersten optischen Datenübertragungsanschluss positioniert ist, wenn das jeweilige Modul (150) für medizinische Vorrichtungen an der induktiven Rückwand (120) gesichert ist

3. System nach Anspruch 2, wobei die Kommunikationsschnittstellen (124) einen Kommunikationsbus (125) umfassen, der mit jedem optischen Daten-Sendeempfänger (126) gekoppelt ist.

4. System nach Anspruch 1, wobei die Kommunikationsschnittstellen (124) mit der induktiven Rückwand (120) gekoppelt sind und die Steuereinheit (140) konfiguriert ist, um über ein induktionsbasiertes Kommunikationsprotokoll die mindestens eine Eigenschaft jedes Moduls (150) für medizinische Vorrichtungen zu steuern, wenn das jeweilige Modul (150) für medizinische Vorrichtungen an der induktiven Rückwand (120) gesichert ist.

5. System nach einem der vorstehenden Ansprüche, wobei jedes Modul (150) für medizinische Vorrichtungen keinen elektrischen galvanischen Verbinder umfasst.

6. System nach einem der vorstehenden Ansprüche, wobei die Vielzahl von Montagesitzen (112) entlang einer vertikalen Achse der Induktor-Rückwand (120) angeordnet ist.

7. System nach einem der vorstehenden Ansprüche, das weiter zu jedem Montagesitz (112) einen entsprechenden Näherungssensor (127) umfasst, wobei der Näherungssensor (127) konfiguriert ist, um das Vorhandensein eines der Vielzahl von Modulen (150) für medizinische Vorrichtungen in dem entsprechenden Montagesitz (112) zu erkennen.

8. System nach Anspruch 7, wobei die mindestens eine Kommunikationsschnittstelle (124) konfiguriert ist, um Kommunikation mit einem der Vielzahl der Module (150) für medizinische Vorrichtungen in einem bestimmten Montagesitz (112) einzuleiten, nachdem der entsprechende Näherungssensor (127) das Vorhandensein des jeweiligen Moduls (150) für medizinische Vorrichtungen in dem Montagesitz (112) erkennt.

9. System nach Anspruch 7 oder 8, wobei die induktive Rückwand (120) so konfiguriert ist, dass sie mit der induktiven Stromversorgung eines der Vielzahl von Modulen (150) für medizinische Vorrichtungen in einem bestimmten Montagesitz (112) beginnt, nachdem der entsprechende Näherungssensor (127) das Vorhandensein des jeweiligen Moduls (150) für medizinische Vorrichtungen in dem Montagesitz (112) erkennt.

10. System nach einem der vorstehenden Ansprüche, weiter umfassend: eine zusätzliche Stromquelle (156) zur Stromversorgung eines oder mehrerer der Vielzahl von Modulen (150) für medizinische Vorrichtungen, die nicht induktiv mit Strom versorgt werden.

11. System nach einem der vorstehenden Ansprüche, weiter umfassend:
eine Touchscreen-Anzeige (130); und
einen verstellbaren Anzeigearm, der die Touchscreen-Anzeige (130) mit der induktiven Rückwand (120) koppelt.

12. System nach Anspruch 11, weiter umfassend:
ein Gehäuse, das die induktive Rückwand (120) umschließt;
wobei die Touchscreen-Anzeige (130) in eine Außenoberfläche des Gehäuses integriert ist.

13. System nach einem der vorstehenden Ansprüche, wobei jedes der Vielzahl von abnehmbaren Modulen (150) für medizinische Vorrichtungen mindestens einen Induktor (1122) und mindestens einen ersten Sensor (1113) umfasst, der zum Erfassen mindestens eines ersten Parameters des mindestens einen Induktors (1122) konfiguriert ist.

14. System nach Anspruch 13, wobei die mindestens eine Kommunikationsschnittstelle (124) eine serielle Brücke einschließt, die für den Austausch der mindestens einen Eigenschaft und des ersten Parameters zwischen der Steuereinheit (140) und mindestens einem der Vielzahl von abnehmbaren Modulen (150) für medizinische Vorrichtungen konfiguriert ist.

15. System nach Anspruch 13 oder 14, wobei die Steuereinheit (140) so konfiguriert ist, dass sie den Stromverbrauch einer oder mehrerer Komponenten eines der Vielzahl von abnehmbaren Modulen (150) für medizinische Vorrichtungen reduziert, wenn der erste Parameter der abnehmbaren Module (150) für medizinische Vorrichtungen einen ersten Grenzwert überschreitet.

## Revendications

1. Système (110) comprenant :
une pluralité de modules de dispositif médical détachables (150), et
un fond de panier inductif (120) configuré pour fixer et alimenter de façon inductive la pluralité de modules de dispositif médical détachables (150) comprenant au moins une pompe de perfusion de fluide (150A, 150B), dans lequel le fond de panier inductif comprend une pluralité de sièges de montage (112), chaque siège de montage étant configuré pour mécaniquement fixer l'un de la pluralité de modules de dispositif médical (150) ;
le fond de panier inductif (120) inclut, pour chaque siège de montage (112), un émetteur inductif (122) pour l'alimentation sans contact de l'un de la pluralité de modules de dispositif médical (150), dans lequel un récepteur inductif (152) correspondant sur le module de dispositif médical (150) respectif est, lorsque le module de dispositif médical (150) respectif est attaché au siège de montage (112), couplé de manière inductive à l'émetteur inductif (122) du fond de panier inductif (120) de telle sorte que l'énergie est envoyée via un couplage inductif entre l'émetteur inductif (122) et le récepteur inductif (152) ;
une interface de communications (124) respective pour chaque siège de montage (112) ; et
une unité de commande (140) pour commander, via l'au moins une interface de communications (124), au moins un attribut de chaque module de dispositif médical (150) lorsque le module de dispositif médical (150) respectif est fixé au fond de panier inductif (120),
**caractérisé en ce que**
chaque siège de montage (112) inclut une étagère avec un élément en queue d'aronde s'étendant à partir d'un boîtier du système (110) configuré pour interagir avec un mécanisme de verrouillage correspondant sur un bord arrière supérieur du module de dispositif médical (150) respectif pour fixer le module de dispositif médical (150) respectif au boîtier de telle sorte que dans l'état fixé le mécanisme de verrouillage est configuré pour réduire la charge sur l'étagère et amener le module de dispositif médical (150) respectif à pivoter pour revenir en contact avec le système (110) sous charge pour s'assurer que l'émetteur inductif (122) est positionné correctement et fixé relativement au récepteur à induction (152).

2. Système selon la revendication 1, comprenant en outre :
au moins un émetteur-récepteur optique de données (126) couplé à l'au moins une interface de communications (124) ; et
une pluralité de premiers ports de transmission de données optiques (124) le long du fond de panier inductif (120) couplé à l'au moins une interface de communications (124) ;
dans lequel chaque module de dispositif médical (150) comprend un second port de transmission de données optiques (154) positionné le long d'un trajet optique avec un premier port de transmission de données optiques correspondant lorsque le module de dispositif médical (150) respectif est fixé dans le fond de panier inductif (120).

3. Système selon la revendication 2, dans lequel les interfaces de communications (124) comprennent un bus de communications (125) couplé à chaque émetteur-récepteur optique de données (126).

4. Système selon la revendication 1, dans lequel les interfaces de communications (124) sont couplées au fond de panier inductif (120) et l'unité de commande (140) est configurée pour commander, via un protocole de communications basé sur l'induction, l'au moins un attribut de chaque module de dispositif médical (150), lorsque le module de dispositif médical (150) respectif est fixé au fond de panier inductif (120).

5. Système selon l'une quelconque des revendications précédentes, dans lequel aucun module de dispositif médical (150) ne comprend de connecteur galvanique électrique.

6. Système selon une quelconque revendication précédente, dans lequel la pluralité de sièges de montage (112) sont agencés le long d'un axe vertical du fond de panier d'inducteur (120).

7. Système selon une quelconque revendication précédente, comprenant en outre un capteur de proximité (127) correspondant à chaque siège de montage (112), le capteur de proximité (127) étant configuré pour détecter la présence de l'un de la pluralité de modules de dispositif médical (150) dans le siège de montage correspondant (112).

8. Système selon la revendication 7, dans lequel l'au moins une interface de communications (124) est configurée pour initier des communications avec l'un de la pluralité de modules de dispositif médical (150) dans un siège de montage particulier (112) après que le capteur de proximité (127) correspondant détecte la présence du module de dispositif médical (150) respectif dans le siège de montage (112).

9. Système selon la revendication 7 ou 8, dans lequel le fond de panier inductif (120) est configuré pour commencer l'alimentation inductive de l'un de la pluralité de dispositif médical (150) dans un siège de montage particulier (112) après que le capteur de proximité (127) correspondant detecte la présence du module de dispositif médical (150) respectif dans le siège de montage (112).

10. Système selon l'une quelconque des revendications précédentes, comprenant en outre : une source d'alimentation auxiliaire (156) pour alimenter un ou plusieurs de la pluralité de modules de dispositif médical (150) qui ne sont pas alimentés par induction.

11. Système selon l'une quelconque des revendications précédentes, comprenant en outre :
un affichage à écran tactile (130) ; et
un bras d'affichage ajustable couplant l'affichage à écran tactile (130) avec le fond de panier inductif (120).

12. Système selon la revendication 11, comprenant en outre :
un boîtier enveloppant le fond de panier inductif (120) ;
dans lequel l'affichage à écran tactile (130) est intégré dans une surface extérieure du boîtier.

13. Système selon l'une quelconque des revendications précédentes, dans lequel chacun de la pluralité de modules de dispositif médical détachables (150) comprend au moins un inducteur (1122) et au moins un premier capteur (1113) configuré pour détecter au moins un premier paramètre de l'au moins un inducteur (1122).

14. Système selon la revendication 13, dans lequel l'au moins une interface de communications (124) inclut un pont sérique configuré pour échanger l'au moins un attribut et le premier paramètre entre l'unité de commande (140) et au moins l'un de la pluralité des modules de dispositif médical détachables (150).

15. Système selon la revendication 13 ou 14, dans lequel l'unité de commande (140) est configurée pour réduire la consommation énergétique d'un ou plusieurs composants de l'un de la pluralité de modules de dispositif médical détachables (150), lorsque le premier paramètre desdits modules de dispositif médical détachables (150) dépasse une première limite.
